# EUROPÄISCHE PATENTSCHRIFT

(11) **EP 2 214 572 B1**
(45) Veröffentlichungstag und Bekanntmachung des Hinweises auf die Patenterteilung: **25.09.2013**
(21) Anmeldenummer: 08854939.9
(22) Anmeldetag: 10.11.2008
(51) Int. Cl.: A61B 17/17, A61B 17/68, A61B 17/16, A61B 17/72

(54) **KNOCHENNAGEL FÜR DIE FERSE**
BONE NAIL FOR THE HEEL
CLOU ORTHOPÉDIQUE POUR LE TALON

(30) Priorität: 26.11.2007 CH 18252007; 27.12.2007 CH 20102007
(43) Veröffentlichungstag der Anmeldung: 11.08.2010
(73) Patentinhaber: Biedermann Motech GmbH & Co. KG, 78054 Villingen-Schwenningen (DE)
(72) Erfinder: Klaue, Kaj, 6942 Savosa (CH)
(74) Vertreter: Lusuardi, Werther
(86) Internationale Anmeldenummer: PCT/CH2008/000474
(87) Internationale Veröffentlichungsnummer: WO 2009/067831

(56) Entgegenhaltungen:
- WO-A-90/12550
- WO-A-03/017822
- WO-A-2006/119659
- WO-A-2007/053960
- WO-A-2007/120539
- US-B1- 6 579 293

## Beschreibung

Die Erfindung bezieht sich auf einen Knochennagel für die tibio-calcaneare Arthrodese gemäss dem Oberbegriff des Patentanspruchs 1, auf einen Fräserführungsdorn für die tibio-calcaneare Arthrodese gemäss dem Oberbegriff des Anspruchs 7, sowie auf ein Osteosynthese-Kit gemäss dem Oberbegriff des Patentanspruchs 16.

Marknägel, welche in einem Teilbereich eine Krümmung aufweisen sind schon lange bekannt. Beispielhaft können hier die sogenannten ENDER-Nägel angeführt werden. Allerdings sind diese bekannten Nägel aufgrund ihres Verwendungszweckes elastisch und flexibel ausgebildet, so dass sie sich schon bei geringen Belastungen durchbiegen. Wegen der Flexibilität dieser bekannten Nägel ist es auch gleichgültig, dass der Mittelteil geradlinig ist und keine Krümmung aufweist. Die bekannten ENDER-Nägel zeichnen sich weiter durch ihre verhältnismässige Länge und Dünne aus. Das Verhältnis zwischen Nageldurchmesser zur Nagellänge liegt bei ENDER im Bereich von 1:60 bis 1:110. Eine Verwendung im Fersenbereich scheidet auch aus diesem Grunde aus.

Aus der WO 2007/120539 HUROWITZ ist ein Marknagel bekannt, der im Wesentlichen gerade und nur an zwei Stellen geknickt ist, so dass der Nagel in zwei verschiedenen Richtungen gekrümmt ist.

Aus der WO 2006/119659 Klaue ist ein Knochennagel mit einer nicht weiter definierten Steifigkeit bekannt, welcher aus einem bioresorbierbaren Material besteht und dessen Länge zweckmässigerweise maximal 60 mm beträgt.

Im Weiteren sind auch Tibia-Nägel bekannt, welche teilweise gekrümmt sind, allerdings sind die Krümmungsradien dieser bekannten Nägel vergleichsweise klein, nämlich im Bereich von 100 und 120 mm, so dass sie für eine Anwendung im Fersenbereich ebenfalls nicht in Frage kommen.

Hier will die Erfindung Abhilfe schaffen. Der Erfindung liegt die Aufgabe zugrunde einen Knochennagel für die tibio-calcaneare Arthrodese zu schaffen, der dank seiner Form und seiner Materialeigenschaften durch den Calcaneus und Talus bis in den Markraum der Tibia einführbar ist. Medizinisch handelt es dabei um eine sogenannte tibio-calcaneare Arthrodese, welche manchmal auch "double arthrodese" genannt wird.

Die Erfindung löst die gestellt Aufgabe mit einem Knochennagel, welcher die Merkmale gemäss Anspruch 1 aufweist, einem Fräserführungsdorn der die Merkmale des Anspruchs 7 aufweist , sowie einem Osteosynthese-Kit, welches die Merkmale des Anspruchs 16 aufweist.

Die wesentlichen Vorteile, die sich mit dem erfindungsgemässen Knochennagel und dank der damit möglichen Operationstechnik erzielen lassen sind die folgenden:
- Möglichkeit der hochgenauen Einstellung des Rückfusses im Verhältnis zur Unterschenkel- und Knieachse;
- Vermeidung des Risikos einer Verletzung der Plantar-Nerven; sowie
- eine weniger invasive Implantation im Vergleich zu anderen Techniken, welche mit Nägeln gemäss dem Stand der Technik angewendet werden müssen.

Dank dem erfindungsgemässen Knochennagel sind nun auch neuartige Indikationen behandelbar, wie z.B. die Behandlung von:
- diabetischen Füssen;
- schmerzhaften Arthrosen des oberen und unteren Sprunggelenkes;
- Nekrose des Talus; und
- Patienten mit Durchblutungsstörungen.

Bei einer besonderen Ausführungsform weist der Knochennagel einen Krümmungsradius von höchstens 210 mm auf. Typischerweise beträgt der Krümmungsradius ca. 190 mm. Zweckmässigerweise variiert der Krümmungsradius über die gesamte Länge des Knochennagels nicht mehr als um 1 % und ist vorzugsweise konstant. Alternativ kann der Krümmungsradius aber auch im vorderen Teil zur Spitze des Knochennagels hin vorzugsweise stetig zunehmen.

Der Fräserführungsdorn ist über seine gesamte Länge steif ausgebildet Diese Ausgestaltung ist am zweckmässigten.
Der vordere Teil des Knochennagels ist weniger steif ausgebildet als der hintere Teil und vorzugsweise plastisch deformierbar. Diese Ausgestaltung ist am zweckmässigsten.
Im vorderen Teil des Knochennagels können auch Längsrillen vorgesehen sein, so dass der vordere Teil einen kleineren Querschnitt aufweist als der hintere Teil. Durch die Schwächung des Querschnitts ergibt sich eine Flexibilisierung des vorderen Teils gegenüber dem steifen hinteren Teil, was bei der Ausführungsform als Implantat Vorteile bietet Der Marknagel kann auch in der Spitzenhälfte rohrförmig ausgestaltet sein, vorzugsweise mit einer Wanddicke von 0,5 mm.
Der vordere Teil weist zweckmässigerweise höchsten 50 % der Steifigkeit des hinteren Teils auf.
Sowohl der vordere Teil als auch der hintere Teil des Knochennagels können 30 bis 70 % der Gesamtlänge des Knochennagels ausmachen.

In einer Ausführungsform weist der Knochennagel einen Durchmesser D₁ von 9 bis 13 mm auf. In einer anderen Ausführungsform ist weist der Fräserführungsdorn einen Durchmesser D_{D} im Bereich von 3 bis 7 mm auf.

Der Knochennagel ist vorzugsweise biegesteif und kann beispielsweise aus einem gehärteten Stahl bestehen. Die Biegesteifigkeit B berechnet sich gemäss Wikipedia nach der Formel: B = E x I , wobei
**I** das Flächenmoment 2. Grades in mm⁴ und E das E-Modul in N / mm² ist.
I ist abhängig von der gewählten Biegeachse; spielt aber bei rotationssymmetrischen Querschnittsflächen keine Rolle.

Der Krümmungsradius R des Knochennagels liegt zweckmässigerweise im Bereich von 140 bis 240 mm, vorzugsweise von 150 bis 210 mm. Diese Bereiche für den Krümmungsradius gestatten ein optimales Einbringen der Knochenbohrung über den Fräserführungsdorn und - nach erfolgter Auffräsung - ein optimales Einbringen des Knochennagels in die drei betroffenen Knochen: Calcaneus, Talus und Tibia. Vorteilhafterweise ist der Krümmungsradius R über die gesamte Länge des Knochennagels konstant. Da sowohl der Knochennagel als auch der Fräserführungsdom steif ist und sich somit kaum verbiegen kann, ist die konstante Krümmung vorteilhaft, um den Knochennagel, bzw. den Fräserführungsdorn mit möglichst geringem Aufwand in die betroffenen Knochen einschlagen zu können.

Der Knochennagel ist zweckmässigerweise über seine gesamte Länge, vorzugsweise stetig, gekrümmt. Diese Ausgestaltung führt zu einer möglichst leichten Einführung des Knochennagels: Ein Knochennagel mit einem gerader Abschnitt (neben gekrümmten Abschnitten) würde die Einführung durch Anstossen des Knochennagels an der Bohrungswand erschweren.

Bei einer besonderen Ausführungsform weist der Knochennagel eine Länge L₁ kleiner als 160 mm, vorzugsweise kleiner als 150 rnm auf. Bei dieser Länge reicht das Implantat bis zu einem Bereich der Tibia, wo dank der Weite des Markkanals ein gewisser Ausgleich stattfinden kann; bei einer grösseren Nagellänge würde die Spitze des Knochennagels in einen weiter verengten Bereich des Tibia-Markkanals gelangen, was die Einführung erschweren würde. Die Länge L_{I} ist aber zweckmässigerweise grösser als 125 mm, vorzugsweise grösser als 130 mm.

Bei einer besonderen Ausführungsform erstreckt sich der Knochennagel über einen Kreisbogen von 38° - 70°, vorzugsweise von 48°- 55°.

Bei einer anderen Ausführungsform weist der Fräserführungsdom eine Länge L_{D} im Bereich von 220 bis 260 mm auf.

Der Knochennagel weist zweckmässigerweise ein Verhältnis L_{I}/D_{I}) zwischen seiner Länge L_{I} und seinem Durchmesser D_{I} auf, welches kleiner als 16, vorzugsweise kleiner als 14 ist. Der Fräserführungsdorn weist zweckmässigerweise ein Verhältnis L_{D}/D_{D} zwischen seiner Länge L_{D} und seinem Durchmesser D_{D} im Bereich von 30 - 90 auf.

Bei einer besonderen Ausführungsform weist der Knochennagel mindestens eine Querbohrung zur Aufnahme einer Verriegelungsschraube auf, wobei die Längsachse der Querbohrung vorzugsweise in der Krümmungsebene des Knochennagels liegt. Die Querbohrung kann z.B. im Kopfbereich des Knochennagels liegen und dessen Längsachse kann einen Winkel alpha von vorzugsweise 10° bis 20° mit der Längsachse des Knochennagels einschliessen.

Bei einer besonderen Ausführungsform weist der Fräserführungsdorn eine scharfe Spitze auf, vorzugsweise in Form einer Meisselspitze. Die Meisselspitze ist vorteilhafterweise nur auf einer Seite abgeschrägt, vorzugsweise auf der Aussenseite der Krümmung des Fräserführungsdorns.

In einer weiteren Ausführungsform beträgt der Krümmungsradius des Knochennagels oder des Fräserführungsdorn mindestens 190 mm, vorzugsweise mindestens 200 mm.

In einer anderen Ausführungsform weist der Knochennagel mindestens im vorderen Teil einen Längsschlitz auf.

In einer weiteren Ausführungsform weist der Knochennagel mindestens im vorderen Teil ein kleeblattförmiges Querschnittsprofil auf.

In einer weiteren Ausführungsform umfasst der Knochennagel zwischen dem vorderen Teil und dem hinteren Teil einen intermediären Teil, dessen Steifigkeit gegen den hinteren Teil zunimmt.

In einer anderen Ausführungsform umfassst der Knochennagel mindestens im vorderen Teil einen zur Längsachse parallelen Hohlraum.

Mindestens ein Knochennagel zusammen mit mindestens einem Fräserführungsdom können zu einem Osteosynthese-Kit zusammengefasst werden, wobei die Krümmungsradien des Knochennagels und des Fräserführungsdoms gleich gross sein müssen. Die Länge L_{D} des Fräserführungsdoms in einem solchen Kit ist vorteilhafterweise 80 bis 120 mm, vorzugsweise 90 bis 110 mm länger als die Länge L_{I} des Knochennagels.

Die Erfindung und Weiterbildungen der Erfindung werden im Folgenden anhand der teilweise schematischen Darstellungen mehrerer Ausführungsbeispiele noch näher erläutert.

Es zeigen:
Fig. 1 eine schematische Schräg-Ansicht der Unterschenkel- und Fuss-Knochen;
Fig. 2 eine Ausführungsform des in die Knochen implantierten erfindungsgemässen Knochennagels;
Fig. 3 eine schematische Ansicht des erfindungsgemässen Knochennagels nach Fig. 2 ;
Fig. 4 eine schematische Ansicht des erfindungsgemässen Fräserführungsdorn;
Fig. 5 den im Knochen implantierten Knochennagel gemäss den Fig. 2 und 3 von schräg vorne und seine Verriegelung mit drei Verriegelungsschrauben;
Fig. 6 die Lage des implantierten Knochennagels gemäss den Fig. 2 und 3 von plantar her gesehen;
Fig. 7 eine schematische Ansicht von antero-lateral einer weiteren Ausführungsform des in den Knochen implantierten erfindungsgemässen Knochennagels;
Fig. 8 einen Querschnitt bei der Linie X - Y in Fig. 7;
Fig. 9 einen Querschnitt bei der Linie Z - Y in Fig. 7; und
Fig. 10 einen Querschnitt bei der Linie W - Y in Fig. 7.

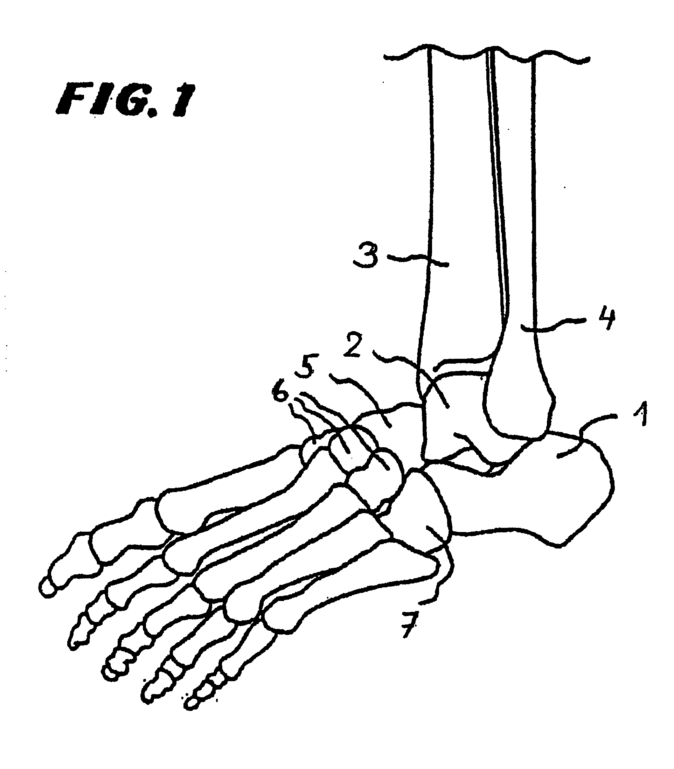

Die Fig. 1 zeigt den Unterschenkel in einer Position, in welcher er um ca. 45° nach Innen gedreht ist Es sind diejenigen Knochen dargestellt, welche für die Anwendung des efindungsgemässen Knochennagels von Bedeutung sind. Es handelt sich primär um den Calcaneus 1, den Talus 2 und die Tibia 3. Die ebenfalls dargestellte Fibula 4, Os naviculare 5, Ossa cuneiformia 6 und Os cuboideum 7 sind nur der Vollständigkeit halber eingezeichnet

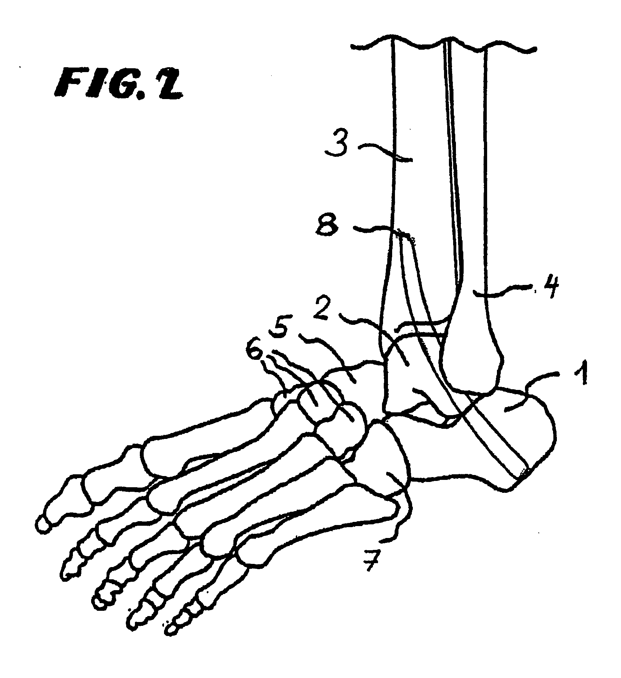

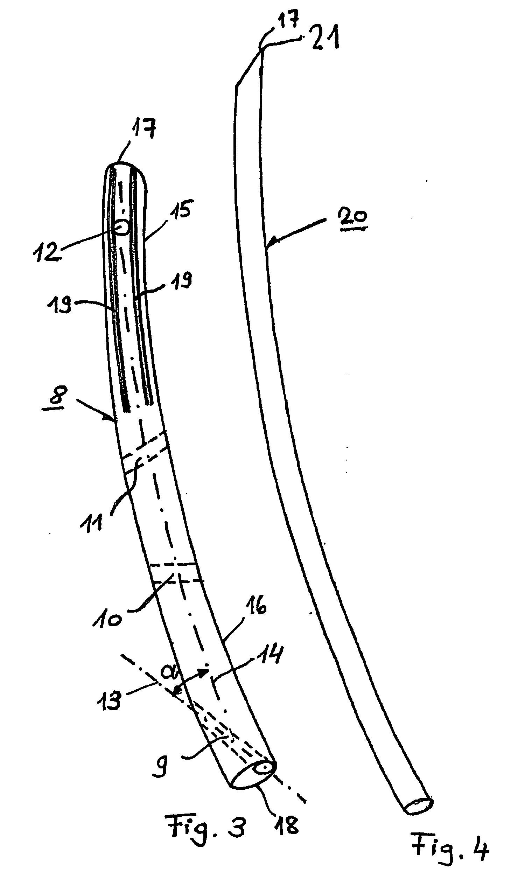

In Fig. 2 ist der erfindungsgemässe als Implantat 8 ausgebildete Knochennagel eingezeichnet, welcher durch den Calcaneus 1 und den Talus 2 bis in den unteren Teil der Tibia 3 implantiert ist. Wie in Fig. 3 dargestellt ist das Implantat 8 in einer Ebene stetig gekrümmt mit einem Krümmungsradius R von typischerweise 190 mm. Die Krümmung erstreckt sich über die gesamte Länge L_{I} des Implantats 8, welche typischerweise 140 mm beträgt. Sein Durchmesser D_{I} beträgt typischerweise 11 mm. Das Verhältnis L_{I}/D_{I} zwischen Länge L_{I} und Durchmesser D_{I} des Implantats 8 beträgt typischerweise 12,7.
Das Implantat 8 weist eine Anzahl von Querbohrungen 9, 10, 11, 12 auf, in welche Verriegelungsschrauben 30 eingeführt werden können. Drei der Querbohrungen 9, 11, 12 liegen in der gleichen Ebene in welcher das Implantat 8 gekrümmt ist. Eine vierte Querbohrung 12 liegt am proximalen Ende des Implantats 8 und verläuft senkrecht zur erwähnten Krümmungsebene.
Die Längsachse 13 der am distalen Ende des Implantats 8 liegenden Querbohrung 9 schliesst mit der Längsachse 14 des Implantats 8 einen Winkel alpha von 15° ein. Das Implantat 8 besteht aus einem steifen Material - insbesondere bezüglich Biegung - und besteht beispielsweise aus einem der bekannten Implantatstähle, welche vorzugsweise gehärtet worden sind, um die Biegesteifigkeit des Implantats 8 weiter zu erhöhen.
Um das Implantat 8 in den Knochen einführen zu können ist es je nach Knochenqualität vorteilhaft die betroffenen Knochen 1, 2, 3 zuvor aufzubohren. Dies geschieht mittels des in Fig. 4 dargestellten Fräserführunsdorn 20 für eine Knochenfräse. Der Fräserführungsdorn 20 muss notwendigerweise die gleiche - in einer Ebene liegende - Krümmung aufweisen wie der als Implantat 8 ausgebildete Knochennagel und ebenfalls aus einem steifen Material bestehen. Er ist aber länger ausgebildet - typischerweise etwa 100 mm länger - als der als Implantat 8 ausgebildete Knochennagel und notwendigerweise dünner als letzterer, typischerweise beträgt sein Durchmesser ungefähr 5 mm.
Der Fräserführungsdorn 20 besitzt ein scharf ausgebildete Spitze 17 in Form einer Meisselspitze 21 um leicht in den Calcaneus 1 einschlagbar zu sein. Die Meisselspitze 21 ist nur auf einer Seite abgeschrägt und zwar auf der Aussenseite der Krümmung des Fräserführungsdoms 20.

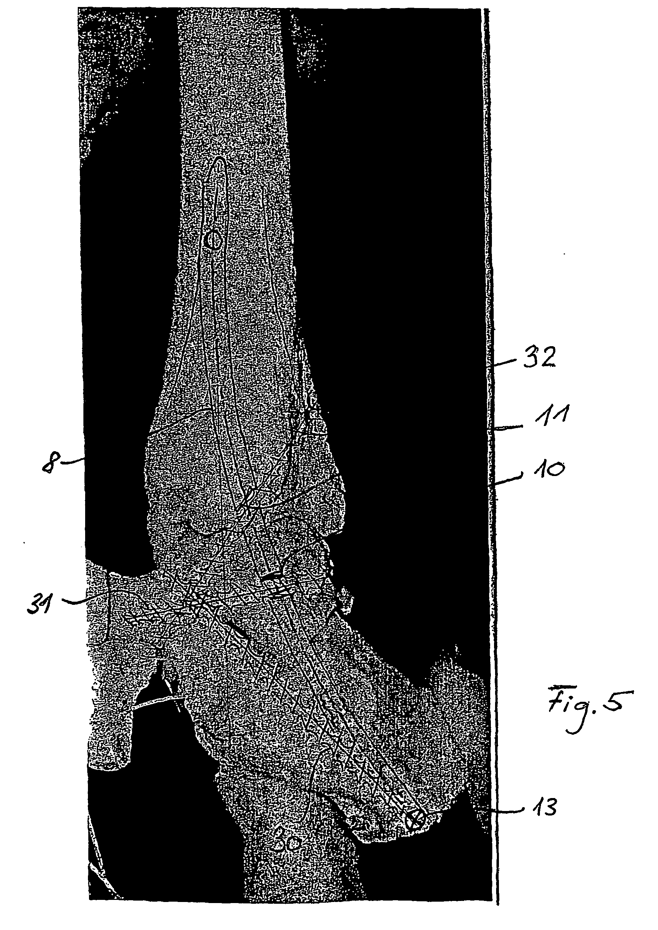

In Fig. 5 ist der Fuss mit dem Unterschenkel um ca. 45° nach Innen gedreht. Das Implantat 8 ist in seiner endgültigen implantierten Lage dargestellt. Eine erste Verriegelungsschraube 30 in der Querbohrung 9 sichert das Implantat 8 im Calcaneus 1 und im Talus 2. Eine zweite Verrieglungsschraube 31 in der Querbohrung 10 blockiert das Implantat 8 im Talus 2. Eine dritte Verrieglungsschraube 32 in der Querbohrung 11 blockiert das Implantat 8 in der Tibia 3 und im Talus 2. Somit werden die drei Knochen Tibia 3, Talus 2 und Calcaneus 1 zusammen versteift und zwar - was wichtig ist - in einer anatomisch richtigen Stellung.

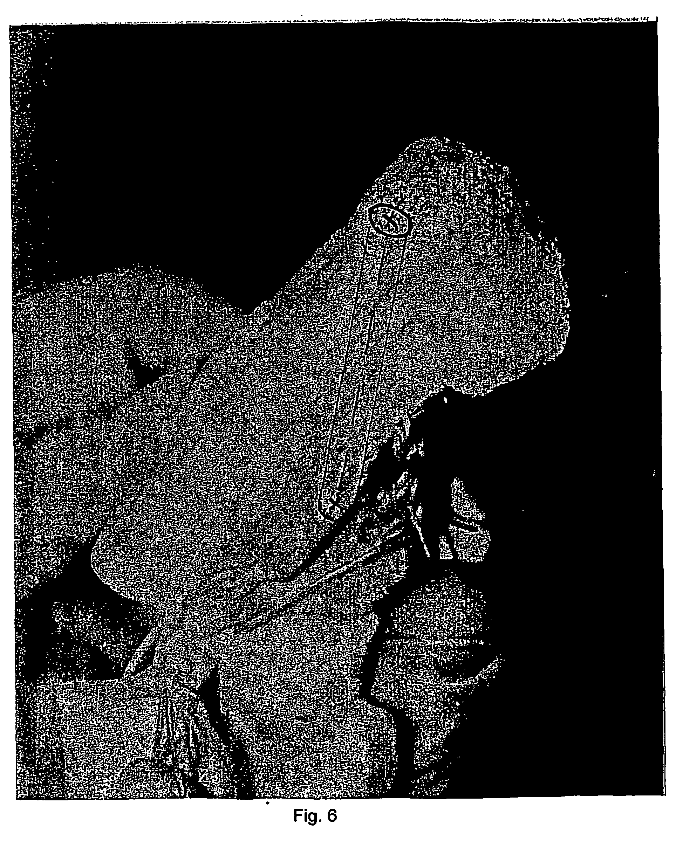

Fig. 6 zeigt einen linken Rückfuss (Skelett) mit der Lage des Eintritt-Orts des erfindungsgemässen Knochennagels und dessen Verlauf im Knochen in einer plantaren Ansicht.

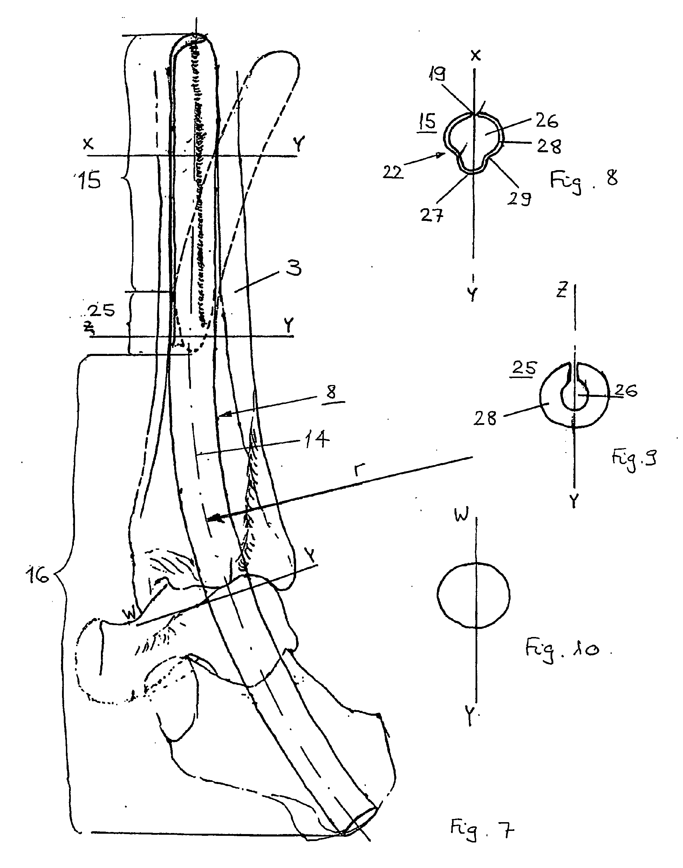

In den Fig. 7 - 10 ist eine weitere Ausführungsform des Knochennagels dargestellt, welche sich von den Ausführungsformen gemäss den Fig. 2 - 6 nur darin unterscheidet, dass der Knochennagel:
a) in seinem vorderen Teil 15 ein kleeblattförmiges Querschnittsprofil 22 (Fig. 8), einen zur Längsachse 14 parallelen Hohlraum 26 und einen zur Längsachse 14 parallelen Längsschlitz 19 umfasst; und
b) zwischen seinem vorderen Teil 15 und seinem hinteren Teil 16 einen intermediären Teil 25 umfasst, welcher als Übergangsbereich zwischen dem vorderen Teil 15 mit kleiner Steifigkeit und dem hinteren Teil 16 mit hoher Steifigkeit ausgebildet ist, so dass die Steifigkeit des Knochennagels gegen den hinteren Teil 16 kontinuierlich zunimmt.

Durch den Hohlraum 26 erhält der Knochennagel im vorderen Teil 15 ein dünnwandiges Querschnittsprofil 22 mit einer peripheren eine geringe Wandstärke aufweisenden Wand 28, welche zudem durch den Längsschlitz 19 parallel zur Längsachse 14 durchtrennt ist. Die Kleeblattform im Querschnittsprofil 22 ergibt sich durch die Ausbildung der Wand 28 mit zwei sich parallel zur Längsachse 14 erstreckenden Einbuchtungen 29 im vorderen Teil 15. Die zwei Einbuchtungen 29 sind derart auf dem Umfang des Knochennagels angeordnet, dass eine dem Längsschlitz 19 diametral gegenüberliegende, sich ebenfalls parallel zur Längsachse erstreckende konvexe Ausbuchtung 27 der Wand 28 gebildet wird. Im intermediären Teil 25 nimmt die Steifigkeit vom vorderen Teil 15 gegen den hinteren Teil 16 durch eine gegen den hinteren Teil 16 zunehmende Wandstärke der Wand 28 zu. Diese zunehmende Wandstärke wird einerseits durch ein kontinuierliches Verengen des Hohlraums 26 und andererseits dadurch erreicht, dass die radiale gemessene Tiefe der Einbuchtungen 29 in der Wand 28 gegen den hinteren Teil 16 kontinuierlich abnimmt, so dass ein kontinuierlicher Übergang vom kleeblattförmigen Querschnittsprofil 22 im vorderen Teil 15 zum kreisförmigen Querschnittsprofil 24 im hinteren Teil 16 gebildet wird. Vor der Implantation hat der intermediäre Teil 25 und der vordere Teil 15 des Knochennagels eine gekrümmte Form (gestrichelt dargestellt) mit demselben Krümmungsradius r wie der hintere Teil 16. Durch die geringere Steifigkeit des intermediären Teils 25 und insbesondere des vorderen Teils 15 können sich diese während des Einbringens in die Tibia 3 deformieren, so dass der vordere Teil 15 nach der Implantation einen erheblich grösseren Krummungsradius aufweisen kann oder sogar geradlinig geformt sein kann.

Im Folgenden wird die generelle Operationsmethode definiert:
1. Entknorpelung der Gelenke und Mobilisation der drei Knochen: Tibia 3; Talus 2 und Calcaneus 1.
2. Einstellung der gewünschten Position der oben erwähnten drei Knochen bezüglich Winkelstellung und Verschiebung.
3. Fixation der oben erwähnten drei Knochen bezüglich ihrer relativen eingestellten Position mittels des als Implantat 8 ausgebildeten Knochennagels; und
4. Optionale Verriegelung des Implantats 8 mit den Verrieglungsschrauben 30.

Im Folgenden wird eine etwas ausführlichere Operationstechnik für den Knochennagel beschrieben:
a) Patient in Rücken-, Bauch-, oder Seitenlage bringen;
b) durch einen gezielten, begrenzten, vorderen, Seiten-, oder postero-lateralen Zugang werden beide Gelenke (oberes und subtalares) angegangen und der Restknorpel entfernt;
c) Tibia 3, Talus 2 und Calcaneus 1 sollen frei beweglich sein um die gewünschte Reorientierung (Einstellung) zu erreichen;
d) die angezielte Position wird mit perkutan eingebrachten, festen Kirschnerdrähten (3 mm Durchmesser) fixiert;
e) Zugang von ca. 2 - 3 cm Länge an der lateralen Ferse;
f) Aufsuchen des postero-lateraten Calcaneusrandes (tuber calcanei);
g) Aufbohren der Corticalis (dünn) mit einem 5 - 7 mm Bohrer;
h) Einführen in den Calcaneus 1 eines gebogenen, an seiner Spitze einseitig geschliffenen erfindungsgemässen Knochennagels in seiner Ausgestaltungsform als Fräserführungsdorn 20 (Durchmesser 6 mm) in einer schrägen Ebene zum Rückfuss (ca. 45° von postero-lateral, gesehen von der Sagittal- oder Frontalebene) [Siehe Fig. 6];
i) Durchquerung des Calcaneus 1 und des Talus 2 auf Höhe der posterioren subtalaren Gelenksfazette;
j) Proximale Durchquerung des Talus 2 auf Höhe des Doms;
k) Die Metaphyse der Tibia 3 wird aufgefädelt, bis dass der Fräserführungsdorn 20 auf der postero-lateralen Corticalis anstösst;
l) der Fräser (Durchmesser 11 mm) wird auf den Fräserführungsdom 20 aufgefädelt und der Weg für den als Implantat 8 ausgebildeten Knochennagel gefräst;
m) Herausziehen des Fräsers und des Fräserführungsdoms 20;
n) Einbringen des Implantats 8 mit eventuellem Zielgriff, der Bohrungen für die Verrieglungsschrauben 30 enthält;
o) Einbringen perkutan der eventuellen Verrieglungsschrauben 30 (für die Tibia 3, den Talus 2, und den Calcaneus 1); und
p) Anbringen der Hautnaht.

## Patentansprüche

1. Knochennagel für die tibio-calcaneare Arthrodese mit
A) einem vorderen zur Einführung in den Knochen bestimmten Teil (15) mit einer Spitze (17); und
B) einem hinteren Teil (16) mit einem Ende (18),
**dadurch gekennzeichnet, dass**
C) der hintere Teil (16) auf einer Länge von mindestens 120 mm, vom Ende (18) aus gerechnet, steif ist, so dass sich der hintere Tell (16) während der Einführung in den Knochen kaum deformiert;
und der vordere Teil (15) weniger steif ist, so daß er sich während des Einbringens in die Tibia deformieren kann.
D) der Knochennagel in einer Ebene gekrümmt ist;
E) die Krümmung für Jeden infinitesimale Abschnitt des Knochennagels einen Krümmungsradius aufweist, der mindestens 130 mm beträgt;
F) der Krümmungsradius höchstens 240 mm beträgt; und
G) der Knochennagel nur in einer Richtung gekrümmt ist.

2. Knochennagel nach Anspruch 1, **dadurch gekennzeichnet, dass** der Krümmungsradius höchstens 210 mm beträgt.

3. Knochennagel nach Anspruch 1 oder 2, **dadurch gekennzeichnet, dass** der Krümmungsradius im vorderen Teil (15) zur Spitze (17) hin vorzugsweise stetig zunimmt.

4. Knochennagel nach einem der Ansprüche 1 bis 3, **dadurch gekennzeichnet, dass** der vordere Teil (15) weniger steif ist als der hintere Teil (16), so dass sich der vordere Teil (15) während des Einbringens in den Knochen deformieren kann und dass der vordere Teil (15) vorzugsweise plastisch deformierbar ausgebildet ist.

5. Knochennagel nach einem der Ansprüche 1 bis 4, **dadurch gekennzeichnet, dass** er als implantat (8) ausgebildet ist und das Verhältnis L_{I}/D_{I} zwischen seiner Länge L_{I} und selnem Durchmesser D_{I} kleiner als 16, vorzugsweise kleiner als 14 ist.

6. Knochennagel nach einem der Ansprüche 1 bis 5, **dadurch gekennzeichnet, dass** er als Implantat (8) ausgebildet ist und mindestens eine Querbohrung (9;10;11;12) zur Aufnahme einer Verriegetungsschraube (30) aufweist, wobei die Längsachse der Querbohrung vorzugsweise in der Krümmungsebene des Knochennagels liegt.

7. Fräserführungsdorn (20) für die tibio-calcaneare Arthrodese mit
A) einem vorderen zur Einführung in den Knochen bestimmten Teil (15) mit einer Spitze (17); und
B) einem hinteren Teil (16) mit einem Ende (18),
**dadurch gekennzeichnet, dass**
C) der hintere Teil (16) auf einer Länge von mindestens 120 mm, vom Ende (18) aus gerechnet, steif ist, so dass sich der hintere Teil (16) während der Einführung in den Knochen kaum deformiert;
und daß der vordere Teil ebenfalls steif ausgebildet ist,
D) der Fräserführungsdorn (20) in einer Ebene gekrümmt ist;
E) die Krümmung für jeden infinitesimalen Abschnitt des Knochennagels einen Krümmungsradius autweist, der mindestens 130 mm beträgt;
F) der Krümmungsradius höchstens 240 mm beträgt;
G) der Fräserführungsdorn (20) nur in einer Richtung gekrümmt ist: und
H) der Fräserführungedorn (20) einen Durchmesser D_{D} im Bereich von 3 bis 7 mm aufweist.

8. Fräserführungsdorn (20) nach Anspruch 7, **dadurch gekennzeichnet, dass** das Verhältnis L_{D}/D_{D} zwischen seiner Länge L_{D} und seinem Durchmesser D_{D} im Bereich von 30 - 90 liegt.

9. Fräserführungsdorn (20) nach Anspruch 7 oder 8, **dadurch gekennzeichnet, dass** die Spitze (17) scharf ausgebildet ist, vorzugsweise In Form einer Meisseispitze (21).

10. Knochennagel nach einem der Ansprüche 1 bis 6, **dadurch gekennzeichnet, dass** der Krümmungsradius mindestens 190 mm beträgt.

11. Knochennagel nach Anspruch 10, **dadurch gekennzeichnet, dass** der Krümmungsradius mindestens 200 mm beträgt.

12. Knochennagel nach einem der Ansprüche 1 bis 6 oder 10 bis 11, **dadurch gekennzeichnet, dass** er mindestens im vorderen Teil (15) einen Längsschlitz (19) aufweist.

13. Knochennagel nach einem der Ansprüche 1 bis 6 oder 10 bis 12, **dadurch gekennzeichnet, dass** er mindestens im vorderen Teil (15) ein kleeblattförmiges Querschnittsprofil (22) aufweist.

14. Knochennagel nach einem der Ansprüche 1 bis 6 oder 10 bis 13, **dadurch gekennzeichnet, dass** der Knochennagel zwischen dem vorderen Teil (15) und dem hinteren Teil (16) einen intermediären Teil (25) umfasst, dessen Steifigkeit gegen den hinteren Teil (16) zunimmt.

15. Knochennagel nach einem der Ansprüche 1 bis 6 oder 10 bis 14, **dadurch gekennzeichnet, dass** der Knochennagel mindestens im vorderen Teil (15) einen zur Längsachse (14) parallelen Hohlraum (26) umfasst.

16. Osteosynthese-Kit umfassend:
a) einen Knochennagel für die tibio-calcaneare Arthrodese nach einem der Ansprüche 1 bis 6 oder 10 bis 15; und
b) einen Fräserführungsdorn (20) nach einem der Ansprüche 7 - 9,
**dadurch gekennzeichnet, dass**
die Krümmungsradien des Knochennagel und des Fräserführungsdorns (20) gleich gross sind.

## Claims

1. Bone nail for the tibio-calcaneal arthrodesis, including:
A) a front part (15) determined for insertion into the bone and having a tip (17); and
B) a rear part (16) having an end (18),
**characterized in that**
C) the rear part (16) is stiff on a length of at least 120 mm starting from the end (18), so that the rear part (16) scarcely deforms during insertion in the bone, and the front part (15) is less stiff, so that it may deform during insertion into the tibia;
D) the bone nail is curved within a plane;
E) the curvature for each infinitesimal section of the bone nail includes a radius of curvature which amounts to at least 130 mm;
F) the radius of curvature amounts to not more than 240 mm; and
G) the bone nail is curved in only one direction.

2. Bone nail according to claim 1, **characterized in that** the radius of curvature amounts to not more than 210 mm.

3. Bone nail according to claim 1 or 2, **characterized in that** the radius of curvature increases preferably steadily in the front part (15) towards the tip (17).

4. Bone nail according to one of the claims 1 to 3, **characterized in that** the front part (15) is less stiff than the rear part (16), so that the front part (15) may deform during insertion into the bone and that the front part (15) is preferably formed being plastically deformable.

5. Bone nail according to one of the claims 1 to 4, **characterized in that** the bone nail is formed as an implant (8) and that the ratio L_{I}/D_{I} between its length L_{I} and its diameter D_{I} is less than 16, preferably less than 14.

6. Bone nail according to one of the claims 1 to 5, **characterized in that** the bone nail is formed as an implant (8) and includes at least one transverse bore (9, 10, 11, 12) for receiving a locking screw (30), wherein the longitudinal axis of the transverse bore preferably lies in the plane of curvature of the bone nail.

7. Milling cutter guiding mandrel (20) for the tibio-calcaneal arthrodesis, including:
A) a front part (15) determined for insertion into the bone and having a tip (17); and
B) a rear part (16) having an end (18),
**characterized in that**
C) the rear part (16) is stiff on a length of at least 120 mm starting from the end (18), so that the rear part (16) scarcely deforms during insertion in the bone, and the front part (15) is stiffly formed as well;
D) the milling cutter guiding mandrel (20) is curved within a plane;
E) the curvature for each infinitesimal section of the milling cutter guiding mandrel (20) includes a radius of curvature which amounts to at least 130 mm;
F) the radius of curvature amounts to not more than 240 mm;
G) the milling cutter guiding mandrel (20) is curved in only one direction; and
H) the milling cutter guiding mandrel (20) has a diameter D_{D} in the range from 3 to 7 mm.

8. Milling cutter guiding mandrel (20) according to claim 7, **characterized in that** the ratio L_{D}/D_{D} between its length L_{D} and its diameter D_{D} lies within the range of 30 - 90.

9. Milling cutter guiding mandrel (20) according to claim 7 or 8, **characterized in that** the tip (17) is formed sharply, preferably in the form of a chisel tip (21).

10. Bone nail according to one of the claims 1 to 6, **characterized in that** the radius of curvature amounts to at least 190 mm.

11. Bone nail according to claim 10, **characterized in that** the radius of curvature amounts to at least 200 mm.

12. Bone nail according to one of the claims 1 to 6 or 10 to 11, **characterized in that** the bone nail comprises at least in its front part (15) a longitudinal slit (19).

13. Bone nail according to one of the claims 1 to 6 or 10 to 12, **characterized in that** the bone nail comprises at least in the front part (15) a clover-leaf shaped cross-section (22).

14. Bone nail according to one of the claims 1 to 6 or 6 to 13, **characterized in that** the bone nail comprises between the front part (15) and the rear part (16) an intermediate part (25), the stiffness of which increases towards the rear part (16).

15. Bone nail according to one of the claims 1 to 6 or 10 to 14, **characterized in that** the bone nail comprises at least in the front part (15) a cavity (26) parallel to the longitudinal axis (14).

16. Osteosynthesis-kit comprising:
a) a bone nail for the tibio-calcaneal arthrodesis according to one of the claims 1 to 6 or 10 to 15; and
b) a milling cutter guiding mandrel (20) according to one of the claims 7 to 9,
**characterized in that**
the radii of curvature of the bone nail and of the milling cutter guiding mandrel (20) have the same size.

## Revendications

1. Clou orthopédique pour l'arthrodèse tibio-calcanéenne avec
A) une partie (15) avant destinée à être introduite dans l'os, avec une pointe (17); et
B) une partie arrière (16) avec une extrémité (18),
**caractérisé en ce que**
C) la partie arrière (16) est rigide sur une longueur d'au moins 120 mm, en comptant à partir de l'extrémité (18), de telle sorte que la partie arrière (16) est à peine déformée pendant l'introduction dans l'os ; et la partie avant (15) est moins rigide, de sorte qu'elle peut se déformer pendant la mise en place dans le tibia ;
D) le clou orthopédique est courbé dans un plan ;
E) la courbure présente, pour chaque tronçon infinitésimal du clou orthopédique, un rayon de courbure qui est au moins égal à 130 mm ;
F) le rayon de courbure est au maximum égal à 240 mm ; et
G) le clou orthopédique n'est courbé que dans une direction.

2. Clou orthopédique selon la revendication 1, **caractérisé en ce que** le rayon de courbure est au maximum égal à 210 mm.

3. Clou orthopédique selon la revendication 1 ou 2, **caractérisé en ce que** le rayon de courbure dans la partie avant (15) augmente, de préférence constamment, en direction de la pointe (17).

4. Clou orthopédique selon une des revendications 1 à 3, **caractérisé en ce que** la partie avant (15) est moins rigide que la partie arrière (16), de telle sorte que la partie avant (15) peut se déformer pendant la mise en place dans l'os, et **en ce que** la partie avant (15) est de préférence réalisée de façon plastiquement déformable.

5. Clou orthopédique selon une des revendications 1 à 4, **caractérisé en ce qu'**il est réalisé en tant qu'implant (8), et **en ce que** le rapport L_{I}/D_{I} entre sa Longueur L_{I} et son diamètre D_{I} est inférieur à 16, de préférence inférieur à 14.

6. Clou orthopédique selon une des revendications 1 à 5, **caractérisé en ce qu'**il est réalisé en tant qu'implant (8) et présente au moins un alésage transversal (9 ; 10 ; 11 ; 12) destiné à recevoir une vis de verrouillage (30), l'axe longitudinal de l'alésage transversal étant de préférence situé dans le plan de courbure du clou orthopédique.

7. Mandrin de guidage de fraise (20) pour l'arthrodèse tibio-calcanéenne avec
A) une partie (15) avant destinée à être introduite dans l'os, avec une pointe (17); et
B) une partie arrière (16) avec une extrémité (18),
**caractérisé en ce que**
C) la partie arrière (16) est rigide sur une longueur d'au moins 120 mm, en comptant à partir de l'extrémité (18), de telle sorte que la partie arrière (16) est à peine déformée pendant l'introduction dans l'os, et **en ce que** la partie avant est également réalisée de façon rigide,
D) le mandrin de guidage de fraise (20) est courbé dans un plan ;
E) la courbure présente, pour chaque tronçon infinitésimal du clou orthopédique, un rayon de courbure qui est au moins égal à 130 mm ;
F) le rayon de courbure est au maximum égal à 240 mm ; et
G) le mandrin de guidage de fraise (20) n'est courbé que dans une direction ; et
H) le mandrin de guidage de fraise (20) présente un diamètre D_{D} dans la plage de 3 à 7 mm.

8. Mandrin de guidage de fraise (20) selon la revendication 7, **caractérisé en ce que** le rapport L_{D}/D_{D} entre sa longueur L_{D} et son diamètre D_{D} se situe dans la plage 30 - 90.

9. Mandrin de guidage de fraise (20) selon la revendication 7 ou 8, **caractérisé en ce que** la pointe (17) est réalisée de façon aiguë, de préférence en forme de pointe de burin (21).

10. Clou orthopédique selon une des revendications 1 à 6, **caractérisé en ce que** le rayon de courbure est au moins égal à 190 mm.

11. Clou orthopédique selon la revendication 10, **caractérisé en ce que** le rayon de courbure est au moins égal à 200 mm.

12. Clou orthopédique selon une des revendications 1 à 6 ou 10 à 11, **caractérisé en ce qu'**il présente au moins dans la partie avant (15) une fente oblongue (19).

13. Clou orthopédique selon une des revendications 1 à 6 ou 10 à 12, **caractérisé en ce qu'**il présente au moins dans la partie avant (15) un profil de section transversale (22) en forme de feuille de trèfle.

14. Clou orthopédique selon une des revendications 1 à 6 ou 10 à 13, **caractérisé en ce que** le clou orthopédique présente entre la partie avant (15) et la partie arrière (16) une partie intermédiaire (25) dont la rigidité augmente en direction de la partie arrière (16).

15. Clou orthopédique selon une des revendications 1 à 6 ou 10 à 14, caractérisé en ce le clou orthopédique comprend au moins dans la partie avant (15) une cavité (26) parallèle à l'axe longitudinal (14).

16. Kit d'ostéosynthèse, comprenant :
a) un clou orthopédique pour l'arthrodèse tibio-calcanéenne selon une des revendications 1 à 6 ou 10 à 15 ; et
b) un mandrin de guidage de fraise (20) selon une des revendications 7 à 9,
**caractérisé en ce que**
les rayons de courbure du clou orthopédique et du mandrin de guidage de fraise (20) sont égaux.
